# EUROPEAN PATENT APPLICATION

(11) **EP 3 392 254 A1**
(43) Date of publication of application: **24.10.2018**
(21) Application number: 17167300.7
(22) Date of filing: 20.04.2017
(51) Int. Cl.: C07F 5/00, A61K 49/00, A61K 51/04

(54) **COMPOUNDS FOR USE IN THE DIAGNOSIS OF A DISEASE**

(71) Applicant: Koninklijke Philips N.V., 5656 AE Eindhoven (NL)
(72) Inventor: PROKSA, Roland, 5656 AE Eindhoven (NL); NOEL, Peter Benjamin, 5656 AE Eindhoven (NL); BUSSE, Madleen, 5656 AE Eindhoven (NL); FINGERLE, Alexander André, 5656 AE Eindhoven (NL)
(74) Representative: de Haan, Poul Erik

(57) **Abstract**

The present invention is directed to a compound comprising lutetium (II) ions, preferably strongly coordinated by organic groups, that is particularly suitable for use as contrast agents in x-ray imaging. The compound inherently provides a safer alternative to known contrast agents and maybe used at lower concentrations as well.

## Description

### FIELD OF THE INVENTION

The present invention generally relates to a compound for use in the diagnosis of a disease, a composition comprising said compound, an imaging method, a method to prepare said compound and a product comprising said compound.

### BACKGROUND OF THE INVENTION

Diagnostic imaging procedures are increasingly performed on a daily bases and help to determine the initial diagnosis and to arrange the treatment and post-treatment. Although the demand and services for magnetic resonance imaging (MRI) and ultrasound procedures are rapidly growing, X-ray imaging techniques, which include computed tomography (CT), digital subtraction angiography (DSA), plain X-ray and X-ray fluoroscopy, remain the driving force of modern radiology.

A drawback of (unenhanced) CT and other x-ray imaging, including CT imaging is that it is limited by reduced contrast of soft tissue, which is important to visualize pathologic structures such as tumors. Contrast agents with enhanced attenuation characteristics compared to particularly soft bodily tissue are used to greatly improve contrast of said tissue, when contrast agent is present in it. Known contrast agents for CT and other x-ray imaging systems are, for example, iodinated and gadolinium-based contrast agents, which have been utilized in a range of applications.

However, use of contrast agents may cause side effects in human or animal subjects. For instance, in some cases severe allergy-like and even fatal reactions were observed after intravenous injection of iodinated contrast media. Other side effects of iodinated contrast agents were observed such as toxic effects on renal cells with contrast-induced kidney injuries observed as complications after intravascular administration of contrast agents.

Because of this there is a need for novel contrast agents for CT and other x-ray imaging methods that have less or less severe side effects.

### SUMMARY OF THE INVENTION

Embodiments according to the present invention are directed to a compound, including any protonated species and any deprotonated species, all isomeric forms, including enantiomers, diastereomers, regioisomers and mixtures thereof, and any pharmaceutically acceptable salt thereof or hydrates thereof, according to the general formula (I), 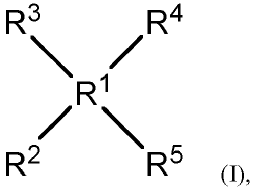 wherein
- R¹ is a linking group chosen from a group comprising an alkyl; alkenyl; alkoxy; alkynyl; cyclic; heterocyclic; aromatic or heteroaromatic group;
- R² is a cyclic or acyclic organic group loaded with a lutetium (III) ion complexed with at least one donor ion;
- each of R³, R⁴ and R⁵ is independently chosen from a group comprising: a cyclic or acyclic organic group loaded with one lutetium (III) ion complexed with at least one donor ion; an alkyl; alkenyl; alkoxy; alkynyl; cyclic; heterocyclic; aromatic; heteroaromatic group or a hydrogen atom.

Said compound is particularly suitable for use in diagnostics of a disease, particularly as a component of a contrast agent or as a contrast agent for x-ray imaging. Lutetium has a smaller atomic radius than other metal ions used in contrast enhancement for diagnostic imaging, for instance, such as known gadolinium based complexes. Due to the smaller radius the lutetium ion may be embedded more stable into a ligand. Therefore the compound will interact less with biological matter, such as organs or blood, increasing the safety of the compound compared to the known compounds used in diagnostics. Preferably, R² has the general structure (II), 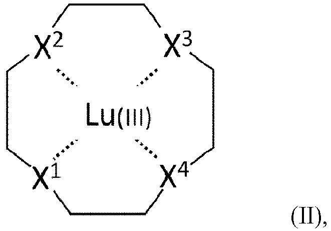 wherein
- X¹, X², X³, X⁴ each is a molecule or group of which at least one of X¹, X², X³, X⁴ comprises at least one donor ion and one of X¹, X², X³, X⁴ is covalently linked to R¹;
- continuous lines represent a covalent bond and dotted lines at least one coordination bond. This provides optimal coordination of the metal ion to the ligand system, making the compound even more stable and therefore safer to use with living biological matter.

Preferably, the donor ion is an oxygen, nitrogen, phosphorous, sulphur ion or combinations thereof. These are common donor ions in coordination chemistry and many options to provide coordination are therewith available.

Preferably, at least one of X¹, X², X³, X⁴ is a group according to the general structure (III),

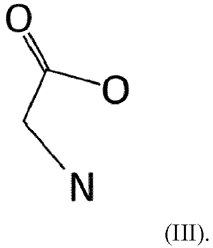

Such a group provides strong coordination through the nitrogen and oxygen molecules, providing a very stable ligand. Also group (III) improves solubility in water, which ensures that the diagnostic agent comprising compound (I) remains only in the arterial system, which eliminates, or at least strongly reduces, leaching into organs or other bodily tissue. The more of group (III) is present in the ligands, the higher the solubility and the better the coordination.

Preferably, R², and preferably at least one of R³, R⁴ and R⁵ comprise a group with the general structure (II') has the general structure (II'),

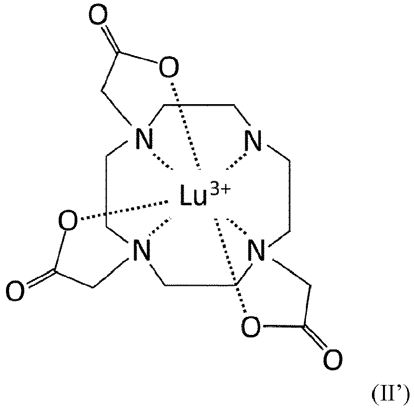

This is a particularly suitable ligand with optimal coordination and high water solubility. If more than one ligand is of this structure, contrast enhancement will be increased further, allowing for a lower dosage compared to known contrast agents, thereby also decreasing the change of side effects and increasing safety to a subject to be imaged. Preferably at least two, and preferably at least three of R², R³, R⁴ and R⁵ comprise a cyclic or acyclic organic group loaded with a lutetium (III) ion complexed with a donor ion, preferably a ligand with structure (II'). This makes compound (I) particularly suitable for overweight subjects, for which traditionally a much higher dosage is required than for average weight subjects.

Preferably, R¹ is a branched or linear alkane or alkene, branched benzene or heterocyclic group or a dentritic structure. Preferably R¹ is a non-ionic moiety. The linking group R¹ may be adapted with the mentioned common organic moieties. These are readily available and it is known in the field how each moiety may influence properties such as solubility and viscosity. A non-ionic moiety reduces viscosity and limits interactions with biological matter even further.

In some preferred embodiments the lutetium (III) ion comprises a radioactive isotope of lutetium (III), preferably ¹⁷⁰Lu, ¹⁷¹Lu, ¹⁷²Lu, ¹⁷⁴Lu, ¹⁷¹Lu, or ¹⁷⁷Lu or combinations thereof. This allows use of compound (I) in radio-diagnostics and radiotherapy applications.

Preferably compound (I) is in a pure or composite particulate form, preferably in a nanoparticulate form. This allows ease of handling and use or as a raw material for further compositions.

Preferably compound (I) comprises or mixed with curable moieties, preferably (co)-polymerizable moieties. This allows for making diagnostic products with compound (I) also outside medical diagnosis, such as coatings or additives in for instance construction materials.

Another embodiment of the present invention is directed towards the compound (I) for use in the diagnosis of a disease. As is explained previously mentioned, compound (I) provides a safer and more effective alternative for use in or as a contrast agent in medical x-ray imaging.

A further embodiment of the present invention is directed towards a composition comprising compound (I). This allows to use compound (I) with other materials to add to their properties or vice versa.

A further embodiment of the present invention is directed towards a method for preparing compound (I) to obtain said diagnostically superior compound compared to known diagnostic compounds.

A further embodiment of the present invention is directed towards a product containing or coated with a compound (I) or a composition comprising compound (I). This allows for imparting the beneficial properties of compound (I) to a product.

Still further aspects and embodiments of the present invention will be appreciated by those of ordinary skill in the art upon reading and understanding the following detailed description. Numerous additional advantages and benefits will become apparent to those of ordinary skill in the art upon reading the following detailed description of preferred embodiments.

### BRIEF DESCRIPTION OF THE DRAWINGS

The present invention is illustrated by drawings of which
Fig. 1 shows structures of preferred ligands for a compound according to the present invention.
Fig. 2 shows schematic drawings of examples of compounds according to the present invention.
Fig. 3 shows structures of preferred examples of compounds according to the present invention.

The invention may take form in various components and arrangements of components, and in various process operations and arrangements of process operations. The drawings are only for the purpose of illustrating preferred embodiments and are not to be construed as limiting the invention. To better visualize certain features may be omitted or dimensions may be not be according to scale.

### DETAILED DESRIPTION OF THE EMBODIMENTS

The presently claimed compounds comprising compound (I) are found to have ideal properties to be used as or in contrast agents in diagnostics obtained through x-ray imaging, including CT. In particular, the compound is suitable for diagnostics involving overweight patients.

The compounds according to the present invention for use in the diagnosis of a disease, have the following general formula (I),

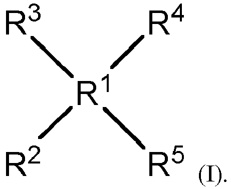

R¹ is a linking group chosen from a group comprising an alkyl; alkenyl; alkoxy; alkynyl; cyclic; heterocyclic; aromatic or heteroaromatic group. Preferably the linking group R¹ is a branched or linear alkane or a branched benzene or heterocyclic group or a dentritic structure. This allows easy installment of more than one lutetium ion in one molecule of compound (I), which improves contrast enhancement compared to the state of the art. Furthermore, it enables installment of a targeting moiety to address a specific biological target, which might involve therapeutic agents (theranostic). Individual components can be easily synthesized or are commercially available. The linking group R¹ allows to install the desired building blocks according to the medical question asked. R² is a ligand comprising a cyclic or acyclic organic group loaded with a lutetium (III) metal ion complexed with a donor ion. Lutetium is the last of the lanthanide metals and it has the smallest atomic radius. It is therefore better suited for the macrocyclic cavity of the ligand and should provide a better complex stability than, for instance, known gadolinium based complexes.

Each of R³, R⁴ and R⁵ are ligands that are independently chosen from a group comprising a cyclic or acyclic organic group loaded with one lutetium (III) metal ion complexed with a donor ion; an alkyl; alkenyl; alkoxy; alkynyl; cyclic; heterocyclic; aromatic; heteroaromatic group or a hydrogen atom. Some or all of R², R³, R⁴ and R⁵ may be the same functional group or all or some may be different from one another. Preferred donor atoms complexing the lutetium (III) ion are oxygen, nitrogen, phosphorous, sulphur atoms or combinations thereof.

The compound (I) includes any protonated species and any deprotonated species, all isomeric forms, including enantiomers, diastereomers, regioisomers and mixtures thereof, and any pharmaceutically acceptable salt thereof or hydrates thereof.

The coordination number of a metal complex refers to the number of ligands (i.e. donor atoms) attached to the metal. For example, in compound (I) of the present disclosure, the lutetium has a coordination number of at least three, for example it may be coordinated by or bonded to at least three groups such as three carboxylate groups. In some embodiments the lutetium may have a higher coordination number, for example it may additionally be coordinated by a neutral molecule such as H₂O. However, in other embodiments the lutetium has a coordination number of three, i.e. compound (I) has a structure consisting of formula (I).

The claimed compound (I) may include salts, solvates, hydrates, isomers, tautomers, racemates, stereoisomers, enantiomers or diastereoisomers of those compounds. Asymmetric centers may exist in the compounds disclosed herein. These centers can be designated by the symbols "R" or "S," depending on the configuration of substituents around the chiral carbon atom. It should be understood that the present disclosure encompasses all stereochemical isomeric forms, including diastereomeric, enantiomeric, and epimeric forms, as well as D-isomers and L-isomers, and mixtures thereof. Additionally, the compounds disclosed herein may exist as geometric isomers. The present invention includes all cis, trans, syn, anti, entgegen (E), and zusammen (Z) isomers as well as the appropriate mixtures thereof. Additionally, compounds may exist as tautomers; all tautomeric isomers are provided by this invention.

Where compound (I) has a net overall charge, for example where the ligand R² to R⁵ complexing the lutetium contains a substituent such as a carboxyl or amino group, compound (I) may be present in the form of a salt. In principle the counter ion may be any organic or inorganic moiety that stabilizes the charge on the parent complex.

Additionally, compound (I) disclosed herein may exist in unsolvated as well as solvated forms. Polymorphic forms of the complexes are also encompassed.

The presently claimed compounds comprising lutetium (III) complexes are designed to provide high water solubility, good thermal stability and good hydrolytic stability, properties which make them particularly suitable for use in a preclinical and/or clinical settings, since leaching and degradation of the compounds over time is minimized.

In some embodiments R³ to R⁵ (hereafter sometimes referred to as R^{a}) independently is C₆₋₁₀ aromatic carbocyclyl or C₅₋₁₀ aromatic heterocyclyl, said aromatic carbocyclyl or aromatic heterocyclyl being unsubstituted or substituted with up to 3 substituents each independently selected from the group consisting of halogen, -CN, -NO₂,-C(O)N(R^{a})₂, -C(O) R^{a}, -S(O)₂N(R^{a})₂, -C(O)O R^{a}; - R^{a}, -O R^{a}, -OC(O) R^{a}, -N(R^{a})₂,-N(R^{a})C(O) R^{a} and -N(R^{a})S(O)₂ R^{a}; and wherein each R^{a} is independently selected from the group consisting of hydrogen, C₁₋₆ alkyl, C₁₋₆ alkenyl, C₁₋₆ alkynyl, C₃₋₁₀ cycloalkyl, C₆₋₁₀ aromatic carbocyclyl and C₅₋₁₀ aromatic heterocyclyl, said alkyl, alkenyl, alkynyl, cycloalkyl, aromatic carbocyclyl or aromatic heterocyclyl being unsubstituted or substituted with up to 3 substituents each independently selected from the group consisting of halogen, -CN, hydroxyl, -C₁₋₆ alkyl, -C₂₋₆ alkenyl, -C₂₋₆ alkynyl, -OC₁₋₆ alkyl, -C₁₋₆ haloalkyl, -NH₂, -NHC₁₋₆ alkyl and -N(C₁₋₆ alkyl)2.

In some embodiments each R^{a} is hydrogen.

In some embodiments each R^{a} is C₁₋₁₀ alkyl which is unsubstituted or substituted with up to 3 substituents each independently selected from the group consisting of halogen, -CN, hydroxyl, -OC₁₋₆ alkyl, NH₂, -NHC₁₋₆ alkyl, -N(C₁₋₆ alkyl)₂ and C₆₋₁₀ aromatic carbocyclyl, which aromatic carbocyclyl is unsubstituted or substituted with up to 3 substituents each independently selected from halogen, -CN, hydroxyl, -C₁₋₆ alkyl, -OC₁₋₆ alkyl and -C₁₋₆ haloalkyl. In some embodiments each R^{a} is C₁₋₆ alkyl which is unsubstituted or substituted with up to 3 substituents each independently selected from the group consisting of halogen, hydroxyl, phenyl, -OC₁₋₄ alkyl, NH₂, -NHC₁₋₄ alkyl and -N(C₁₋₄ alkyl)₂. In some embodiments each R^{a} is C₁₋₆ alkyl. In some embodiments each R^{a} is methyl, ethyl, n-propyl or iso-propyl.

In some embodiments each R^{a} is independently C₆₋₁₀ aromatic carbocyclyl or C₅₋₁₀ aromatic heterocyclyl, said aromatic carbocyclyl or aromatic heterocyclyl being unsubstituted or substituted with up to 3 substituents each independently selected from the group consisting of halogen, -CN, -NO₂ hydroxyl, -C₁₋₆ alkyl, -C₂₋₆ alkenyl, -C₂₋₆ alkynyl,-OC₁₋₆ alkyl, -C₁₋₆ haloalkyl, -NH₂, -NHC₁₋₆ alkyl and -N(C₁₋₆ alkyl)₂. In some embodiments each R^{a} is C₆₋₁₀ aromatic carbocyclyl or C₅₋₁₀ aromatic heterocyclyl, said aromatic carbocyclyl or aromatic heterocyclyl being unsubstituted or substituted with up to 3 substituents selected from the group consisting of halogen, -CN, -NO₂ -C₁₋₆ alkyl, -C₂₋₆ alkenyl, -C₂₋₆ alkynyl and - OC₁₋₆ alkyl. In some embodiments each R^{a} is phenyl which is unsubstituted or substituted with up to 3 substituents each independently selected from the group consisting of halogen, - CN, -NO₂ C₁₋₆ alkyl, -C₂₋₆ alkenyl, -C₂₋₆ alkynyl and -OC₁₋₆ alkyl. In some embodiments R^{a} is a phenyl group which comprises an ortho-substituent. In some embodiments R^{a} is a phenyl group which comprises a meta-substituent. In some embodiments R^{a} is a phenyl group which comprises a para-substituent. In some embodiments R^{a} is phenyl which is unsubstituted or substituted with one -NO₂ group or one -OMe group.

In the structure of formula (I), R² to R⁵ is a ligand bonded to lutetium through a donor atom, which is preferably oxygen, nitrogen, phosphorous, sulphur or a combination thereof. The chelator is cyclic, heterocyclic, aromatic cyclyl or aromatic heterocyclyl group and may be unsubstituted or substituted. Where the aromatic carbocyclyl or aromatic heterocyclyl group is substituted, it is bonded to lutetium through a bond between the lutetium and a donor atom present in the aromatic carbocyclyl or aromatic heterocyclyl ring system. Where the aromatic carbocyclyl or aromatic heterocyclyl group is substituted, it may for example contain 1, 2 or 3 substituents each independently selected from the group consisting of halogen, -CN, -NO₂ -C(O)N(R^{a})₂, -C(O) R^{a}, -S(O)₂N(R^{a})₂, -C(O)O R^{a}; - R^{a}, -O R^{a}, -OC(O) R^{a}, -N(R^{a})₂, -N(R^{a})C(O) R^{a} and -N(R^{a})S(O)₂R^{a}; and wherein each R^{a} is independently selected from the group consisting of hydrogen, C₁₋₆ alkyl, C₁₋₆ alkenyl, C₁₋₆ alkynyl, C₃₋₁₀ cycloalkyl, C₆₋₁₀ aromatic carbocyclyl and C₅₋₁₀ aromatic heterocyclyl, said alkyl, alkenyl, alkynyl, cycloalkyl, aromatic carbocyclyl or aromatic heterocyclyl being unsubstituted or substituted with up to 3 substituents each independently selected from the group consisting of halogen, -CN, hydroxyl, -C₁₋₆ alkyl, -C₂₋₆ alkenyl, -C₂₋₆ alkynyl, -OC₁₋₆ alkyl, -C₁₋₆ haloalkyl, -NH₂, -NH_{C1-6} alkyl and -N(C₁₋₆ alkyl)₂.

In some embodiments the ligand is substituted with a C₆₋₁₀ aromatic carbocyclyl or C₅₋₁₀ aromatic heterocyclyl, said aromatic carbocyclyl or aromatic heterocyclyl being unsubstituted or substituted with up to 3 substituents each independently selected from the group consisting of halogen, -CN, -NO₂ hydroxyl, -C₁₋₆ alkyl, -C₂₋₆ alkenyl, -C₂₋₆ alkynyl, -OC₁₋₆ alkyl, -C₁₋₆ haloalkyl, -NH₂, -NHC₁₋₆ alkyl and -N(C₁₋₆ alkyl)2. In some embodiments each substituent is C₆₋₁₀ aromatic carbocyclyl or C₅₋₁₀ aromatic heterocyclyl, said aromatic carbocyclyl or aromatic heterocyclyl being unsubstituted or substituted with up to 3 substituents selected from the group consisting of halogen, -CN, -NO₂ -C₁₋₆ alkyl, -C₂₋₆ alkenyl, -C₂₋₆ alkynyl and -OC₁₋₆ alkyl. In some embodiments the substituent is phenyl which is unsubstituted or substituted with up to 3 substituents each independently selected from the group consisting of halogen, -CN, -NO₂ C₁₋₆ alkyl, -C₂₋₆ alkenyl, -C₂₋₆ alkynyl and -OC₁₋₆alkyl.

In some embodiments the ligand is substituted with a phenyl group, which is unsubstituted or substituted with 1 or 2 substituents each independently selected from the group consisting of halogen, -CN, -NO₂ -C₁₋₆ alkyl, -C₂₋₆ alkenyl, -C₂₋₆ alkynyl and -OC₁₋₆ alkyl. In some embodiments the substituent is a phenyl group which comprises an ortho substituent. In some embodiments the substituent is a phenyl group which comprises a meta-substituent. In some embodiments the substituent is a phenyl group which comprises a para-substituent. In some embodiments the substituent is unsubstituted phenyl.

In preferred embodiments one to four of ligands R², R³, R⁴ and R⁵ have the general structure (II):

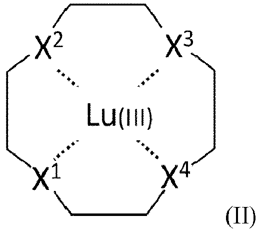

X¹, X², X³, X⁴ each is a molecule or functional group which preferably comprises at least one donor ion and one of X¹, X², X³, X⁴ is covalently linked to R¹ (bond not shown). Continuous lines represent a covalent bond and dotted lines a coordination bond. Preferably two of the other three of X¹, X², X³, X⁴ have the same structure. This provides optimal coordination of the metal ion to the ligand system. Also endogenous compounds such as water, carbonates, phosphonates, etc. maybe involved in the binding to the lutetium ion.

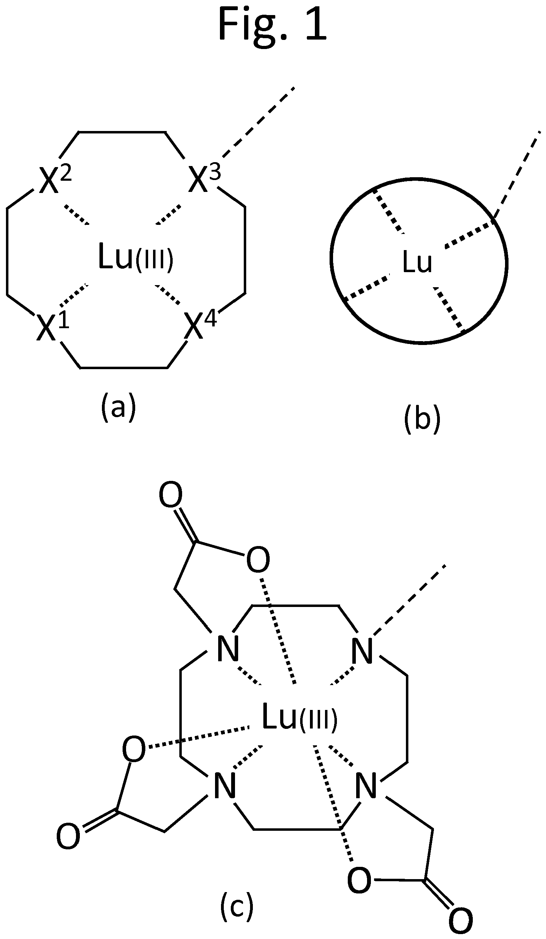

Figure 1a shows the same structure with the addition that a dashed line represents the bond from one of one of X¹, X², X³, X⁴ (shown here for X³) to the linking group R¹. Figure 1b shows a schematic representation of the lutetium (III) complex that is used in further images. While this schematic representation somewhat mimics the general structure (II), it is not to be misunderstood that this schematic representation should only cover the general structure (II), it represents any compound (I) suitable for the present invention.

One or more of X¹, X², X³, X⁴ are preferably N-Y, wherein N is nitrogen and Y is hydrogen or an organic group, preferably an organic group that may contribute to the coordination of the lutetium (III) ion. Most preferably, one of X¹, X², X³, X⁴ is a nitrogen group linking to R¹ and one or more of the others of X¹, X², X³, X⁴ have the general structure (III): 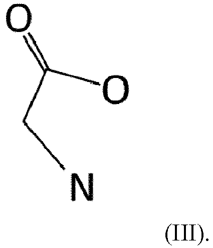 wherein the nitrogen is part of the ring structure (indicated by the dashed lines) and both the nitrogen and the single bonded oxygen coordinate the Lutetium (III) ion (indicated by the dotted lines). Figure 1c shows a preferred example of a compound wherein X¹, X², X³ have the structure (III) and X⁴ is a nitrogen atom that provides a covalent bond to R¹ (indicated by the dashed line) and also contributes to the coordination of the lutetium (III) ion. This structure optimally coordinates the lutetium (III) ion and also increases water solubility of the lutetium-based compounds (I), and therefore, allow for an in vivo application in a preclinical and/or clinical setting. Particularly acyclic ligand systems such as DTPA (diethylenetriaminepentacetic acid) based ligands provide very stable lutetium complexes.

Compounds with one to three of ligands R^{2,} R³, R⁴ and R⁵ according to general structure (II) are preferred because installment of more than one lutetium ion per molecule improves contrast enhancement during x-ray imaging. This may lower the dose of contrast agent for lower weight patients and should supply enough contrast for overweight patients. Most preferred are compounds with three ligands according to general structure (II), because this will provide high contrast enhancement and, furthermore, the Lu metal is a lanthanide metal, which builds complexes with high coordination numbers, usually around 8-9. This means that lutetium can have up to nine ligands. The preferred oxidation state is +3, and therefore, in order to compensate the charge on the metal center, three anionic ligands (total charge of -3) are preferred. The other 6 coordination numbers maybe provided by lone pairs of donor atoms such as nitrogen, oxygen, etc. or even neutral ligands such as water molecules.

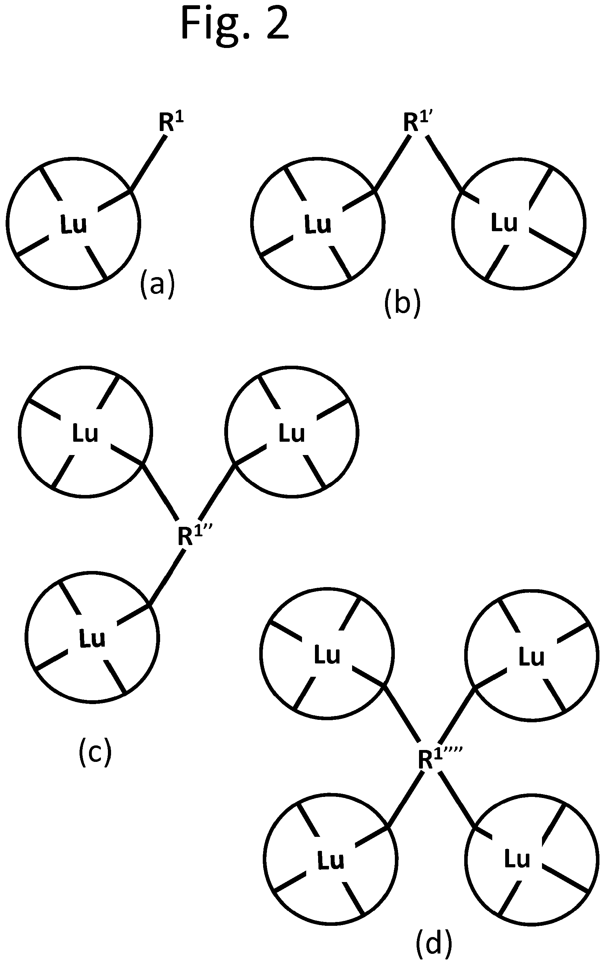

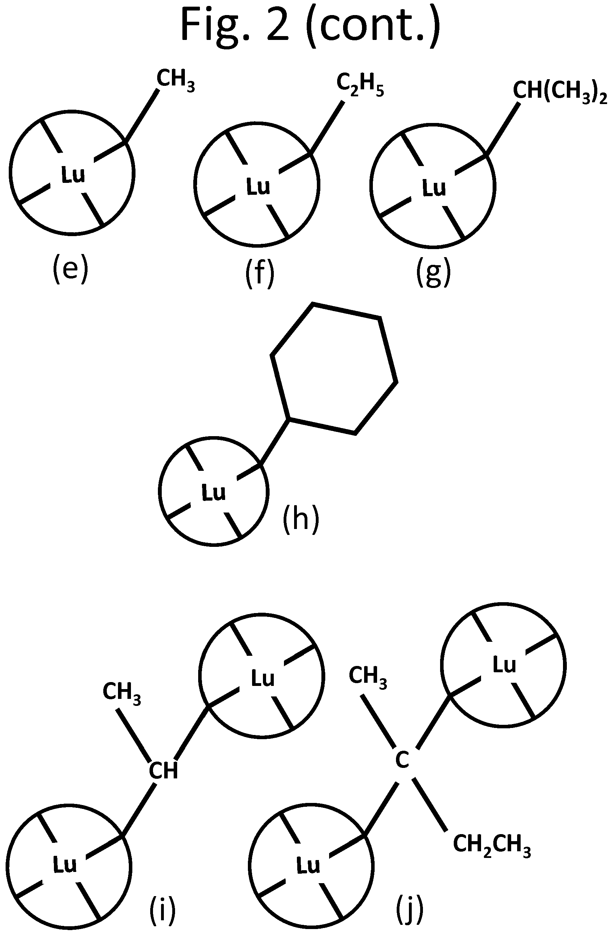

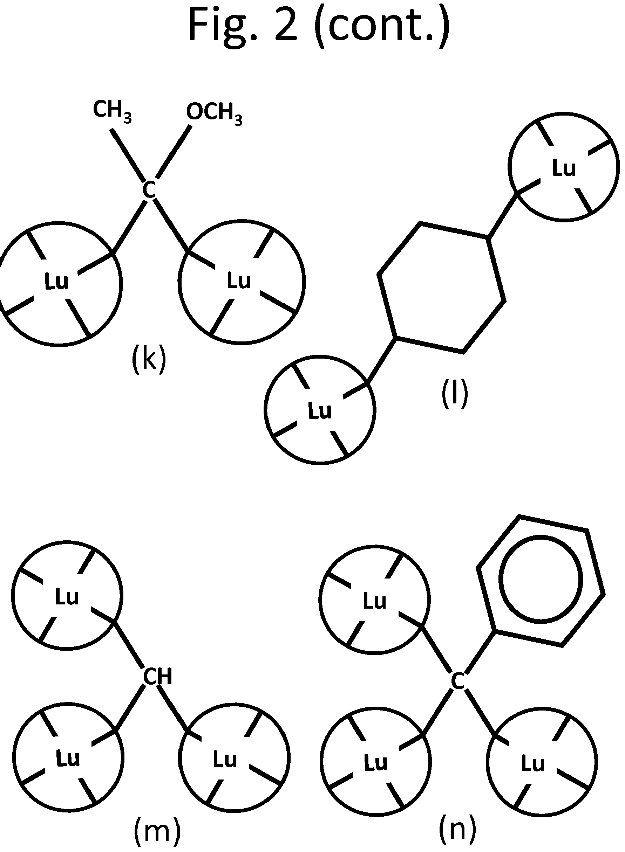

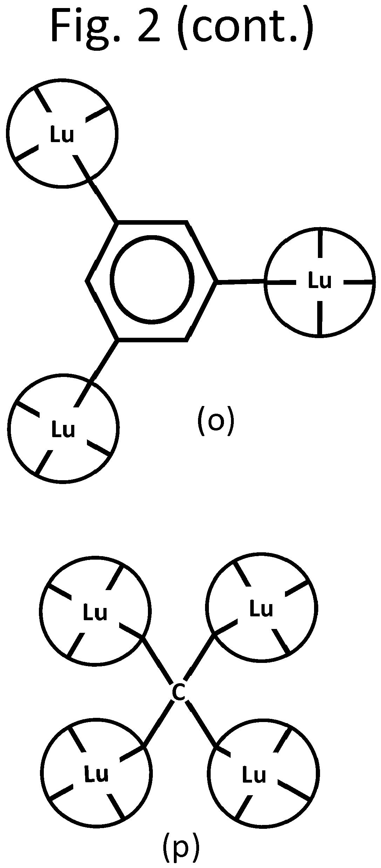

Figure 2 shows a non-limiting number of examples of compounds (I) to illustrate the present invention. These are purely illustrative and non-limiting. Some of the shown examples may be sterically hindered and are therefore not optimal, but are shown to avoid overly cluttered images. Preferably, one to three methyl groups are installed as spacer between the Lu containing sub-units

Figures 2a to 2d show versions of compound (I) with respectively one, two, three and four ligands with structure (II), using the simplified representation of figure 1b. In each of these examples R¹ may be the same (obviously accounting for the number of present ligands) or different.

Figures 2e to 2n show further examples using different amounts of ligands (II) and types of linking groups R¹.

Figures 2e to 2h show compounds (I) with one ligand (II). Figures 2i to 2l show compounds (I) with two ligands (II) which may be on neighboring or opposite sites of the linking group. Figures 2m to 2o show compounds (I) with three ligands (II). Figures 2p shows compound (I) with four ligands (II)

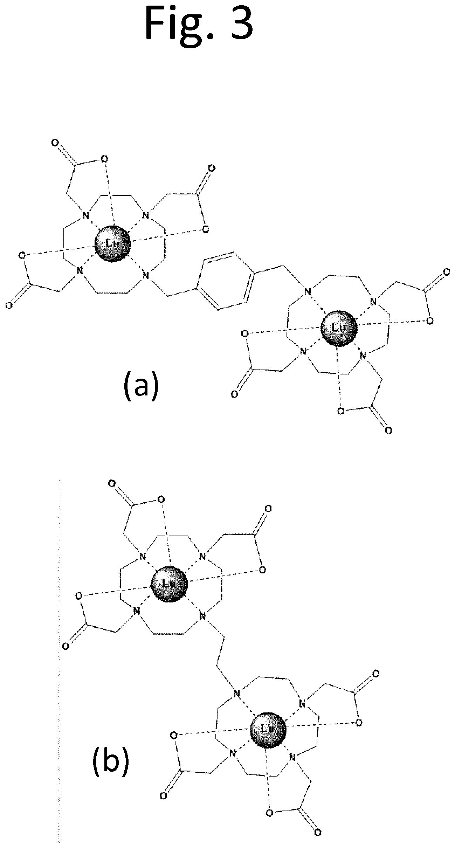

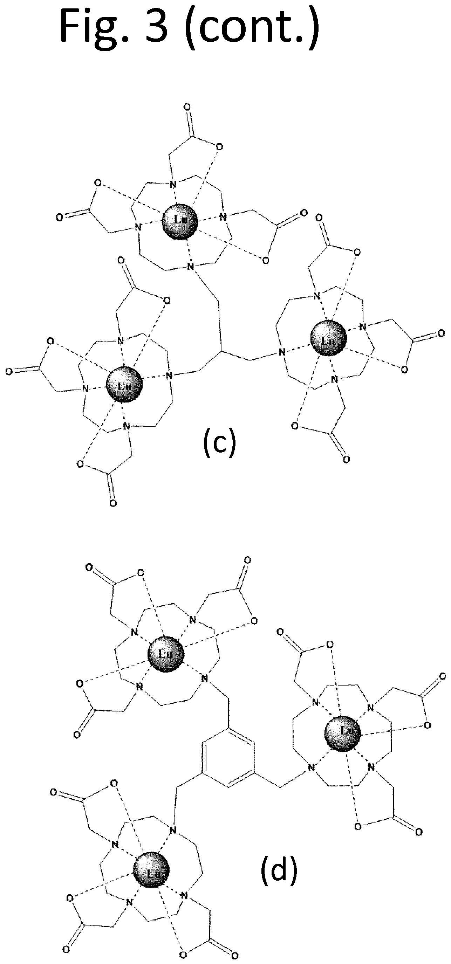

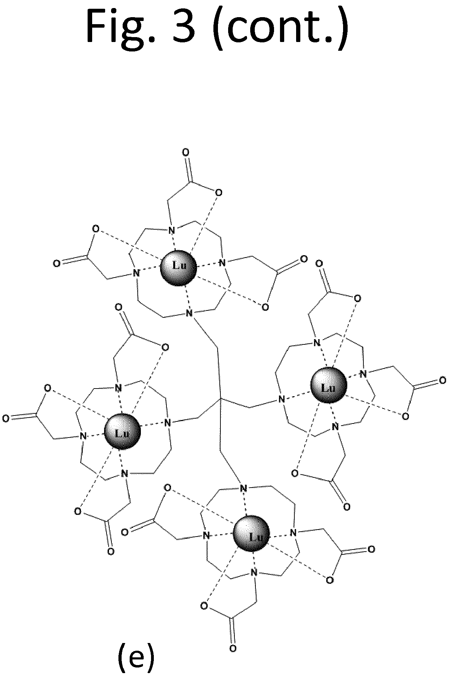

Figures 3a to 3e show examples of compounds (I) with two or more of structure 1c as ligands of the structure shown in figure 1c.

Table 1 shows an overview of R¹ to R⁵ in figures 2e to 2p and figures 3a to 3e. This is a non-limiting list of examples. Any combination of the individual groups mentioned in table 2 in any position is envisioned, as well as any other combination including other organic groups known to a person skilled in the art, such as longer branched and unbranched alkyl groups, other, optionally fused, ring structures, oligomeric or polymeric structures and the like.

**Table 1. Overview of linking groups and ligands of figures 2e to 2p and 3a to 3 e.**

| Figure | R¹ | R² | R³ | R⁴ | R⁵ |
|---|---|---|---|---|---|
| 2e | Carbon atom | Lu-ligand | Hydrogen | Hydrogen | Hydrogen |
| 2f | Carbon atom | Lu-ligand | Methyl | Hydrogen | Hydrogen |
| 2g | Carbon atom | Lu-ligand | Methyl | Hydrogen | Methyl |
| 2h | Cyclohexyl | Lu-ligand | Hydrogen | Hydrogen | Hydrogen |
| 2i | Carbon atom | Lu-ligand | Methyl | Lu-ligand | Hydrogen |
| 2j | Carbon atom | Lu-ligand | Methyl | Lu-ligand | Ethyl |
| 2k | Carbon atom | Lu-ligand | Methyl | Methoxy | Lu-ligand |
| 2l | Cyclohexyl | Lu-ligand | Hydrogen | Lu-ligand | Hydrogen |
| 2m | Carbon atom | Lu-ligand | Lu-ligand | Hydrogen | Lu-ligand |
| 2n | Carbon atom | Lu-ligand | Lu-ligand | Phenyl | Lu-ligand |
| 2o | Benzene ring | Lu-ligand | Lu-ligand | Hydrogen | Lu-ligand |
| 2p | Carbon atom | Lu-ligand | Lu-ligand | Lu-ligand | Lu-ligand |
| 3a | 1,4-Dimethylbenzene | Lu-ligand | Hydrogen | Lu-ligand | Hydrogen |
| 3b | Ethane | Lu-ligand | Hydrogen | Lu-ligand | Hydrogen |
| 3c | Isobutane | Lu-ligand | Lu-ligand | Lu-ligand | Hydrogen |
| 3d | 1,3,5-Trimethylbenzene | Lu-ligand | Lu-ligand | Lu-ligand | Hydrogen |
| 3e | Isobutane | Lu-ligand | Lu-ligand | Lu-ligand | Lu-ligand |

Using different linking groups and types or numbers of Lutetium (III) ligands, may influence x-ray absorption characteristics (more lutetium means more contrast enhancement), viscosity and compatibility with the human body or particular organs. A non-ionic compound is preferred as it keeps the viscosity low and limits interactions on a biological molecular level within the human body, making it more compatible and less toxic.

Use of compound (I) as or in contrast agents is particularly suitable for use in overweight subjects, because the signal strength of a contrast material including lutetium in overweight patient is improved compared with current state of the art contrast agents because of a better balance of photons below and above the k-edge. The installment of more than one lutetium ion in compound (I) improves the contrast enhancement during x-ray imaging. This lowers the overall dose of contrast agent for lower weight patients and should supply sufficient contrast for overweight patients.

The compound (I) according to the present invention may be provided as part of a composition, for instance in a composition with other contrast enhancing materials, solvents, diluents, additives, excipients and other support materials known in the medical field. Preferably the composition is a medical composition with only medically acceptable materials. In some embodiments the compound is distributed within a material, which may be a solid or a liquid. In some embodiments the compound or the composition is polymerizable and/or otherwise curable. The diagnostic properties of compound (I) make them particularly suitable for use in the hospital/medical environment.

The compounds (I) according to the present invention are particularly suitable for use as additives in products, materials and the like where diagnostic properties are desirable. The compounds (I) are stable to both moisture and temperature and have low solubility in organic solvent, but have a high water solubility. The compounds (I) are therefore particularly suitable for use in a preclinical and/or clinical settings, since leaching and degradation of the compound over time is minimized. Compound (I) has high water-solubility, thereby providing a good hydrolytic and redox stability (no trans-metalation of the lutetium ion, which would result in a free Lu³⁺ ion). Due to high contrast enhancement (especially for molecules with more than one lutetium ion), also lower contrast agent doses may be necessary. All these factors individually and together ensure that chemical or physical interaction between body parts and the contrast agent is minimal, thereby reducing potential toxicity and providing a much safer alternative to known contrast agents for x-ray imaging.

In embodiments the compounds (I) have a half-life (i.e. the time taken for 50% of the compound to be converted to a different species) in water at 25°C of at least 24 hours, at least 48 hours, at least 72 hours, more most at least 1 week; at 15°C at least 2 weeks, at least 1 month, at least 2 months, at least 3 months, at least 6 months, or most at least 1 year. In preferred embodiments the compound (I) is thermally stable upon heating to at least 100°C. In preferred embodiments the compound (I) has an aqueous solubility at 25°C of less than 10g/1, less than 1g/1, less than 0.5 g/l, less than 0.2 g/l, less than 0.1 g/l, less than 0.05 g/l, less than 0.02 g/l, or most less than 0.01 g/l.

In further embodiments compound (I) may be present in the form of small particles, e.g. nanoparticles. The compound (I) may be present in the particle in a pure form (i.e. with no other compounds) or in a composite form (i.e. mixed with one or more compounds in any ratio). Diagnostic properties of compound (I) maybe improved in such embodiments because an active surface area is maximized, particularly in cases where solubility of compound (I) is lower than optimal, in order to maximize contact of the compound with a biological target. In further embodiments the compound (I) is present in particulate form wherein the particles have a mean diameter of less than 1000 µm, less than 500 µm, less than 200 µm, less than 100 µm, less than 50 µm, less than 20 µm, less than 10 µm, less than 5 µm, less than 2µm, or most less than 1 µm. In some embodiments the compound (I) is present in particulate form wherein the particles have a mean diameter in the range of from 1000 µm to 500 µm, from 500 µm to 200 µm, from 200 µm to 100 µm, from 100 µm to 50 µm, from 50 µm to 20 µm, from 20 µm to 10 µm, from 10 µm to 5 µm, from 5 µm to 2µm, or most from 2 µm to 1 µm.

The compound (I) may be used as diagnostic additive for coating surfaces of products, or for dispersal within products (e.g. polymeric products) in a variety of applications. Compound (I) retains the same advantages as it would have in the pure form, including being long-lasting, having low levels of degradation and low levels of leaching. Accordingly, the present disclosure also provides a product, device, material or composition comprising compound (I).

In further embodiments compound (I) is distributed within a product, device, material or composition. For example, the composition may be a curable composition. A curable composition is a composition, which is capable of being hardened or fixed, for example by exposure of a composition to high temperature, exposure to light (e.g. UV light) or exposure to atmospheric conditions (e.g. compositions which cure or harden as a result of reaction with moisture present in the atmosphere). In further embodiments, the curable composition may be one which cures by virtue of evaporation of solvent present in the composition. In further embodiments, the curable compositions may be one which cures by virtue of a chemical reaction taking place between components present in the composition (e.g. on exposure to high temperature, or to ultraviolet light) and/or between components present in the composition and substances present in the surrounding environment (e.g. atmospheric water). The curable composition comprising compound (I) may for example be a one-part composition (e.g. a composition which cures/hardens on exposure to atmospheric conditions, UV light, or heat). Alternatively, in cases where the composition is prepared by mixing together multiple precursor compositions (e.g. a first composition comprising polymers or prepolymers, and a second composition comprising cross-linker or hardener) which cure by virtue of a chemical reaction taking place between components present in the different compositions upon admixing, compound (I) may for example be present in one of the precursor compositions. Examples of curable compositions include adhesive compositions, such as cement compositions. Further examples include sealant compositions. The composition may for example be a coating composition for coating the surface of an object, e.g. a composition comprising a film former/binder, compound (I), and optionally other components such as diluent, pigment, and/or filler. The composition may for example be a monomer composition comprising a polymerizable monomer which is intended for polymerization to produce a polymerized, plastic, product, and which contains compound (I) as an additive.

The product, device, material or composition may be a polymeric material comprising compound (I). For example, the compound may be dispersed as a separate component within the bulk of the polymeric material. Alternatively, where for example the polymeric material is produced by carrying out polymerization in the presence of compound (I) which itself may have a polymerizable group such as an alkenyl group (e.g. where at least one of R² to R⁵ is a styrene group), the compound may be present in the polymeric material as an integral part of the polymer (i.e. covalently bound to the polymer).

As an alternative to compound (I) being dispersed within a product, a product may be coated with surface coating comprising compound (I). For example, the exterior of a product may be covered by spraying or coating with the compound or a composition comprising the compound. In further embodiments, for example where compound (I) is for use in a surface coating composition of an object, and where compound (I) has insufficient solubility, the composition may take the form of a suspension composition comprising compound (I) suspended in a liquid carrier.

The amount of compound (I) used in products, compositions and the like depends on the nature of the material and the intended application. In some embodiments, the compound is present in an amount of up to 25, up to 20, up to 15, up to 10, up to 5, up to 2, up to 1, up to 0.5, up to 0.2, up to 0.1, up to 0.05, up to 0.02 up to 0.01, up to 0.005, up to 0.002, or up to 0.001% by weight of the product, material, device or composition which comprises compound (I).

Accordingly, in embodiments the product, device, material or composition comprising compound (I) is a medical product, device, material or composition, such as a wound dressing, a suture, a surgical implement or a medical implant. For example sutures or stitches (e.g. stitches formed of polymers having glycolic acid and/or lactic acid monomer units, such as polygalactin 910) maybe coated with a coating composition comprising compound (I), or the stitches formed from monomer compositions containing the compound. Surgical equipment and implements, and medical implants such as dental implants, stents and components used in joint arthroplasty, may be coated with a coating composition comprising compound (I) or, where appropriate, formed of a composition comprising the compound (I). Compound (I) may also or alternatively find use in wound dressings. For example the product may be a wound dressing comprising a woven material made of synthetic and/or natural polymer (e.g. polyurethane, polyester or cotton) coated or impregnated with a composition comprising compound (I), or, in the case of a synthetic polymer, formed of a monomer composition containing compound (I). The medical product, device, composition or material comprising compound (I) may also be a curable medical adhesive (e.g. a cyanoacrylate composition), a bone or dental cement (e.g. methyl methacrylate / polymethyl methacrylate compositions), a dental primer or dental adhesive. Medical consumables where diagnostic properties are desirable may also comprise compound (I).

Compound (I) may be used to impart diagnostic properties to a variety of medical products. For example wound dressings coated or impregnated with the complex may be applied to the skin of a patient to prevent to monitor the therapeutic outcome, or the diagnostic compound may be present in a bone cement composition to visualize the attachment to the human body following joint replacement surgery. Compound (I) of the present disclosure, and compositions comprising compound (I), find use in the treatment planning and post-treatment actions.

The present invention also provides in a method comprising imaging of a tissue of interest of a subject. The tissue of interest comprises a diagnostically effective amount of a compound (I) according to the present invention. The presently claimed compounds and compositions are very suitable for x-ray imaging including CT imaging, because of high contrast and low interaction with the body, making them safer and more effective. The one or more lutetium ions per molecule of compound (I), which is a highly X-ray absorbing heavy metal, increases the local concentration, which improves the contrast. More lutetium ions per molecule of compound (I).

Accordingly, provided herein is compound (I) as previously defined, or a composition comprising compound (I), for use in diagnostics, therapy planning and/or post-treatment. Also provided herein is a method of diagnostic evaluation of a disease or disorder in a subject, comprising administering to the subject a diagnostically effective amount of compound (I) as defined in above, or of a composition comprising compound (I). Also provided herein is a pharmaceutical agent comprising a complex as defined previously. Where the compound is administered in the form of a composition, such as bone cement or dental cement, the additional components of the composition, such as carrier and/or filler, are selected so as to be acceptable for administration to a patient. Compound (I) disclosed herein find use in the treatment planning and/or post-treatment actions of a disease or disorder in a subject.

The subject to whom compound (I) is administered maybe an animal, for example a mammal. In embodiments, the subject is a human. In further embodiments, the subject is a non-human animal. In some embodiments, compound (I) is not used on a human or animal body, e.g. it is used ex vivo, in or on products and materials in the environment.

Compound (I) may also find use in diagnostic applications outside the medical environment. For example, the complex may find use in products, devices, materials or compositions used in building construction, renovation and/or maintenance. Examples of compositions which may comprise compound (I) as a diagnostic additive include curable and/or polymerizable compositions, such as coating compositions (e.g. lacquer compositions, varnish compositions, or paint compositions comprising a binder or film former, and optionally other components such as pigment and/or diluent), adhesive or sealant compositions (e.g. a silicone or polyurethane sealant composition), cement compositions (e.g. a Portland-type cement composition comprising calcium silicates), concrete (e.g. compositions comprising cement, aggregates and water), or grout, mortar or stucco compositions (e.g. compositions comprising water, cement and sand).

Compound (I) according to the present invention may be prepared by first obtaining a compound according to general structure (IV):

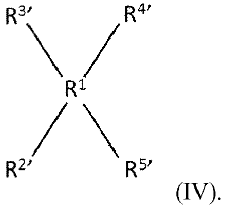

In this structure R¹ is the same as previously defined for compound (I). R^{2,} corresponds to a ligand R² as previously defined for compound (I), but not loaded with a Lu (III) ion, accounting for chemical stability with potentially other atoms stabilizing or capping the complexing groups, e.g. a hydrogen atom, and organic group or a counter ion. R² may preferably be a ligand according to the general structure (V): 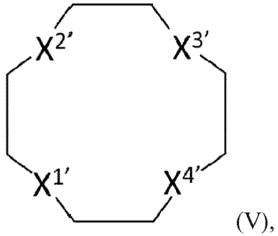 wherein X^{1,}, X², X³ and X⁴ correspond to X¹, X², X³ and X⁴ as previously defined for compound (II), accounting for chemical stability with potentially other atoms stabilizing or capping the complexing groups, e.g. a hydrogen atom, and organic group or a counter ion. R^{3,}, R^{4,}, R^{5,} may corresponds to a ligand R² as previously defined, but not loaded with a Lu (III) ion, accounting for chemical stability with potentially other atoms stabilizing or capping the complexing groups or any of the groups as previously defined for R³, R⁴, R⁵, preferably C₆₋₁₀ aromatic carbocyclyl or C₅₋₁₀ aromatic heterocyclyl, said aromatic carbocyclyl or aromatic heterocyclyl being unsubstituted or substituted with up to 3 substituents each independently selected from the group consisting of halogen, -CN, -NO₂ C₁₋₆ alkyl, -C₂₋₆ alkenyl, -C₂₋₆ alkynyl and -OC₁₋₆ alkyl.

Next, compound (IV) is reacted with a steochioemetrically correct amount of lutetium (III) oxide (Lu₂O₃) to form compound (I)., The stoichiometric correct amount depends, amongst others, on the number of ligands to be complexed with lutetium (III).

This synthetic process yields compound (I) reproducibly in high yield, with no further purification being required in many cases.

In a preferred embodiment one or more of the lutetium (III) molecules in the compound (I) may comprise radioactive isotopes of lutetium. According to the "tracer principle" a radioactive substance participates in an organism's physiological processes in the same way as non-radioactive materials. As such the biological distribution and toxicity profile of the disclosed compound (I) is not affected by changing the lutetium isotope.

Accordingly, the exchange of the different isotopes of lutetium allows for a "matched isotopic pair". The observationally stable isotope ¹⁷⁵Lu with abundance of 98% is active for CT and nuclear magnetic resonance (NMR), and therefore, also suitable for MR Imaging. Isotopes ¹⁷⁰Lu, ¹⁷¹Lu, ¹⁷²Lu, ¹⁷⁴Lu are examples for positron emitting lutetium isotopes. These have relatively long half-lives of 2 days, 8 days, 6.7 days and 3.3 years respectively, which makes them an interesting positron source for positron emission tomography (PET) diagnostics, therapy planning (personalized dosimetry calculations resulting from the diagnostic imaging techniques) and treatment. Isotope ¹⁷⁷Lu is a beta emitter enabling radiotherapy, e.g. used in the treatment of cancer patients, and post-treatment (monitoring and controlling via the diagnostic techniques).

As used herein, the term "alkyl" encompasses straight and branched chain saturated hydrocarbon groups. Examples of alkyl groups include methyl, ethyl, n-propyl, iso-propyl, n-butyl, t-butyl, i-butyl, sec-butyl, pentyl and hexyl groups.

As used herein, the term "alkoxy" means the group O-alkyl, where "alkyl" is used as described above. Examples of alkoxy groups include methoxy and ethoxy groups. Other examples include propoxy and butoxy.

As used herein, the term "alkenyl" encompasses both straight and branched chain unsaturated hydrocarbon groups with at least one carbon-carbon double bond. Examples of alkenyl groups include ethenyl, propenyl, butenyl, pentenyl and hexenyl. Preferred alkenyl groups include ethenyl, 1-propenyl, 2-propenyl and but-2-enyl.

As used herein, the term "alkynyl" encompasses both straight and branched chain unsaturated hydrocarbon groups with at least one carbon-carbon triple bond. Examples of alkynyl groups include ethynyl, propynyl, butynyl, pentynyl and hexynyl. Preferred alkynyl groups include ethynyl, 1-propynyl and 2-propynyl.

As used herein, the terms "halo" or "halogen", whether employed alone or in compound words such as haloalkyl, means fluorine, chlorine, bromine or iodine.

As used herein, the term "haloalkyl" means an alkyl group having at least one halogen substituent, the terms "alkyl" and "halogen" being understood to have the meanings outlined above. Similarly, the term "monohaloalkyl" means an alkyl group having a single halogen substituent, the term "dihaloalkyl" means an alkyl group having two halogen substituents and the term "trihaloalkyl" means an alkyl group having three halogen substituents. Examples of monohaloalkyl groups include fluoromethyl, chloromethyl, bromomethyl, fluoromethyl, fluoropropyl and fluorobutyl groups; examples of dihaloalkyl groups include difluoromethyl and difluoroethyl groups; examples of trihaloalkyl groups include trifluoromethyl and trifluoroethyl groups.

As used herein, the terms "cyclic" and "cyclyl" represent a ring system wherein the ring atoms are all carbon atoms, e.g. from 3 to 20 carbon ring atoms, and which may be aromatic, non-aromatic, saturated, or unsaturated. The terms encompass single ring systems, e.g. cycloalkyl groups such as cyclopentyl and cyclohexyl, aromatic groups such as phenyl, and cycloalkenyl groups such as cyclohexenyl, as well as fused-ring systems such as naphthyl and fluorenyl.

As used herein, the terms "heterocyclic' and "heterocyclyl" represent an aromatic or a non-aromatic cyclic group of carbon atoms wherein from one to three of the carbon atoms is/are replaced by one or more heteroatoms independently selected from nitrogen, oxygen, phosphorous or sulfur. A heterocyclyl group may, for example, be monocyclic or polycyclic, and contain for example from 3 to 20 ring atoms. In a bicyclic heterocyclyl group there may be one or more heteroatoms in each ring, or only in one of the rings. Examples of heterocyclyl groups include piperidinyl, tetrahydrofuranyl, tetrahydropyranyl, pyridyl, pyrimidinyl and indolyl.

As used herein, the term "cycloalkyl" represents a ring system wherein the ring atoms are all carbon atoms, e.g. from 3 to 20 carbon ring atoms, and which is saturated. A cycloalkyl group can be monocyclic or polycyclic. A bicyclic group may, for example, be fused or bridged. Examples of monocyclic cycloalkyl groups include cyclopropyl, cyclobutyl and cyclopentyl. Other examples of monocyclic cycloalkyl groups are cyclohexyl, cycloheptyl and cyclooctyl. Examples of bicyclic cycloalkyl groups include bicyclo[2.2. 1]hept-2-yl.

As will be understood, an "aromatic" group means a cyclic group having 4m+2π electrons, where m is an integer equal to or greater than 1. As used herein, "aromatic" is used interchangeably with "aryl" to refer to an aromatic group, regardless of the valency of aromatic group.

As used herein, the terms "aromatic carbocyclyl" or "aromatic carbocycle" represent a ring system which is aromatic and in which the ring atoms are all carbon atoms, e.g. having from 6-14 ring atoms. An aromatic carbocyclyl group maybe monocyclic or polycyclic. Examples of aromatic carbocyclyl groups include phenyl, naphthyl and fluorenyl. Polycyclic aromatic carbocyclyl groups include those in which only one of the rings is aromatic, such as for example indanyl.

As used herein, the terms "aromatic heterocycle" or "aromatic heterocyclyl" represent an aromatic cyclic group of carbon atoms wherein from one to three of the carbon atoms is/are replaced by one or more heteroatoms independently selected from nitrogen, oxygen or sulphur, e.g. having from 5-14 ring atoms. The term "aromatic heterocyclyl" is used interchangeably with 'heteroaryl". An aromatic heterocyclyl group may be monocyclic or polycyclic. Examples of monocyclic aromatic heterocyclyl groups (also referred to as monocyclic heteroaryl groups) include furanyl, thienyl, pyrrolyl, imidazolyl, pyridyl and pyrimidinyl. Examples of polycyclic aromatic heterocyclyl groups (also referred to as bicyclic heteroaryl groups) include benzimidazolyl, quinolinyl and indolyl. Polycyclic aromatic heterocyclyl groups include those in which only one of the rings is an aromatic heterocycle.

As used herein, the term "cyano" represents a -CN moiety.

As used herein, the term "hydroxyl" represents a -OH moiety.

As used herein, the term "alkoxy" represents an -O-alkyl group in which the alkyl group is as defined supra. Examples include methoxy, ethoxy, n-propoxy, iso-propoxy, and the different butoxy, pentoxy, hexyloxy and higher isomers.

As used herein, the term "aryloxy" represents an -O-aryl group in which the aryl group is as defined supra. Examples include, without limitation, phenoxy and naphthoxy.

As used herein, the term "carboxyl" represents a -CO₂H moiety.

As used herein, the term "nitro" represents a -NO₂ moiety.

As used herein, the term "amino" represents a -NH₂ moiety.

The term "optionally fused" means that a group is either fused to another ring system or unfused, and "fused" refers to one or more rings that share at least two common ring atoms with one or more other rings. Fusing may be provided by one or more carbocyclic or heterocyclic rings, as defined herein, or be provided by substituents of rings being joined together to form a further ring system. The fused ring maybe a 5, 6 or 7-membered ring of between 5 and 10 ring atoms in size. The fused ring may be fused to one or more other rings, and may for example contain 1 to 4 rings.

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or groups of compositions of matter. Thus, as used herein, the singular forms "a", "an" and "the" include plural aspects unless the context clearly dictates otherwise. For example, reference to "a" includes a single as well as two or more; reference to "an" includes a single as well as two or more; reference to "the" includes a single as well as two or more and so forth.

Those skilled in the art will appreciate that the disclosure herein is susceptible to variations and modifications other than those specifically described. It is to be understood that the disclosure includes all such variations and modifications. The disclosure also includes all of the steps, features, compositions and compounds referred to or indicated in this specification, individually or collectively, and any and all combinations or any two or more of said steps or features.

Each example of the present disclosure described herein is to be applied mutatis mutandis to each and every other example unless specifically stated otherwise. The present disclosure is not to be limited in scope by any of the specific examples described herein, which are intended for the purpose of exemplification only. Functionally equivalent products, compositions and methods are clearly within the scope of the disclosure as described herein.

The term "and/or", e.g. "X and/or Y" shall be understood to mean either "X and Y" or "X or Y" and shall be taken to provide explicit support for both meanings or for either meaning.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

## Claims

1. A compound, including any protonated species and any deprotonated species, all isomeric forms, including enantiomers, diastereomers, regioisomers and mixtures thereof, and any pharmaceutically acceptable salt thereof or hydrates thereof, according to the general formula (I), 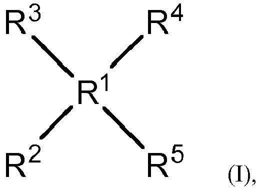 wherein
- R¹ is a linking group chosen from a group comprising an alkyl; alkenyl; alkoxy; alkynyl; cyclic; heterocyclic; aromatic or heteroaromatic group;
- R² is a cyclic or acyclic organic group loaded with a lutetium (III) ion complexed with at least one donor ion;
- each of R³, R⁴ and R⁵ is independently chosen from a group comprising: a cyclic or acyclic organic group loaded with one lutetium (III) ion complexed with at least one donor ion; an alkyl; alkenyl; alkoxy; alkynyl; cyclic; heterocyclic; aromatic; heteroaromatic group or a hydrogen atom.

2. A compound according to claim 1, wherein R² has the general structure (II), 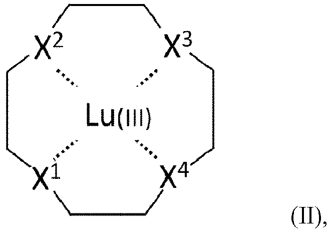 wherein
- X¹, X², X³, X⁴ each is a molecule or group of which at least one of X¹, X², X³, X⁴ comprises at least one donor ion and one of X¹, X², X³, X⁴ is covalently linked to R¹;
- continuous lines represent a covalent bond and dotted lines at least one coordination bond.

3. A compound according to any of the previous claims, wherein the donor ion is an oxygen, nitrogen, phosphorous, sulphur ion or combinations thereof.

4. A compound according to claim 2, wherein at least one of X¹, X², X³, X⁴ is a group according to the general structure (III),

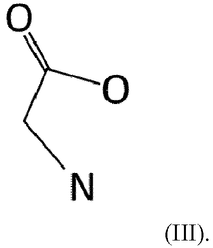

5. A compound according to any of the previous claims, wherein R², and preferably at least one of R³, R⁴ and R⁵ comprise a group with the general structure (II') has the general structure (II'),

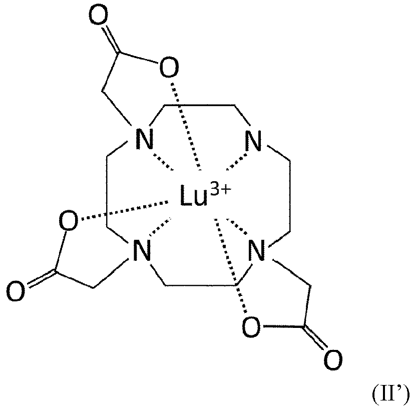

6. A compound according to any of the previous claims, wherein at least two, and preferably at least three of R², R³, R⁴ and R⁵ comprise a cyclic or acyclic organic group loaded with a lutetium (III) ion complexed with a donor ion.

7. A compound according to any of the previous claims, wherein R¹ is a branched or linear alkane or alkene, branched benzene or heterocyclic group or a dentritic structure, most preferably R¹ is a non-ionic moeiety.

8. A compound according to any of the previous claims, wherein the lutetium (III) ion comprises a radioactive isotope of lutetium (III), preferably ¹⁷⁰Lu, ¹⁷¹Lu, ¹⁷²Lu, ¹⁷⁴Lu, ¹⁷⁵Lu, or ¹⁷⁷Lu or combinations thereof.

9. A compound according to any of the previous claims present in a pure or composite particulate form, preferably in a nanoparticulate form.

10. A compound according to any of the previous claims comprising or mixed with curable moieties, preferably (co-)polymerizable moieties.

11. A compound, according to any of the previous claims for use in the diagnosis of a disease.

12. Composition comprising a compound according to any of the previous claims.

13. Method comprising imaging of a tissue of interest of a subject that comprises a diagnostically effective amount of a compound (I) according to any of the claims 1 to 11.

14. Method for preparing a compound according to general structure (I) according to the present invention by:
(i) obtaining a compound according to general structure (IV): 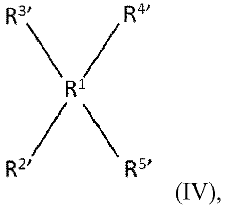
wherein R¹ is the same as defined in claim 1;
R^{2,} corresponds to a ligand R² as defined in claim 1, but not loaded with a Lu (III) ion, accounting for chemical stability with potentially other atoms stabilizing or capping the complexing groups;
R^{3,}, R^{4,}, R^{5,} each individually correspond to a ligand R² as defined in claim 1, but not loaded with a Lu (III) ion or any of the groups as defined in claim 1 for R³, R⁴, R⁵, accounting for chemical stability with potentially other atoms stabilizing or capping the complexing groups.

15. Product containing or coated with a compound (I) according to any of the claims 1 to 11 or with a composition according to claim 12.
